# EUROPEAN PATENT APPLICATION

(11) **EP 3 342 401 A1**
(43) Date of publication of application: **04.07.2018**
(21) Application number: 17210692.4
(22) Date of filing: 27.12.2017
(51) Int. Cl.: A61K 9/20, A61K 31/4439

(54) **BILAYER TABLET FORMULATIONS OF DABIGATRAN ETEXILATE**

(30) Priority: 28.12.2016 TR 201619828; 19.12.2017 TR 201720779
(71) Applicant: Sanovel Ilac Sanayi ve Ticaret A.S., 34460 Istanbul (TR)
(72) Inventor: TÜRKYILMAZ, Ali, 34460 Istanbul (TR); GÜLKOK, Yildiz, 34460 Istanbul (TR); PALANTÖKEN, Arzu, 34398 Istanbul (TR); SÖZER BÜRKÜT, Gökce, 34460 Istanbul (TR)
(74) Representative: Sevinç, Erkan

(57) **Abstract**

The present invention relates to a pharmaceutical bilayer tablet comprising dabigatran etexilate free base or pharmaceutically acceptable salts of dabigatran etexilate and at least one pharmaceutically acceptable excipient.

## Description

### Field of Invention

The present invention relates to a pharmaceutical bilayer tablet comprising dabigatran etexilate free base or pharmaceutically acceptable salts of dabigatran etexilate and at least one pharmaceutically acceptable excipient.

### Background of the Invention

Dabigatran etexilate, a novel direct thrombin inhibitor, is a prodrug of dabigatran and is a non-peptide thrombin inhibitor. It was developed by Boehringer Ingelheim Pharmaceuticals, Inc. The oral drug is absorbed by the gastrointestinal tract and converts to dabigatran which has the direct anticoagulant activity. Dabigatran binds to the specific fibrin-binding sites of thrombin, preventing cleavage of fibrinogen to fibrin and blocking the final steps in the coagulation cascade network and thrombosis. Dabigatran can be dissociated from a fibrin-thrombin complex and play reversible anticoagulant effect.

Chemical name of dabigatran etexilate is 3-[(2-{[4-(hexyloxycarbonylamino-iminomethyl)-pheny-lamino]-methyl}-1-methyl-1H-benzimidazol-5-carbonyl)-pyridin-2-yl-amino] propionic acid ethyl ester, having a formula of C₃₄H₄₁N₇O₅, a molecular weight of 627.74 and a chemical structural formula shown below (I):

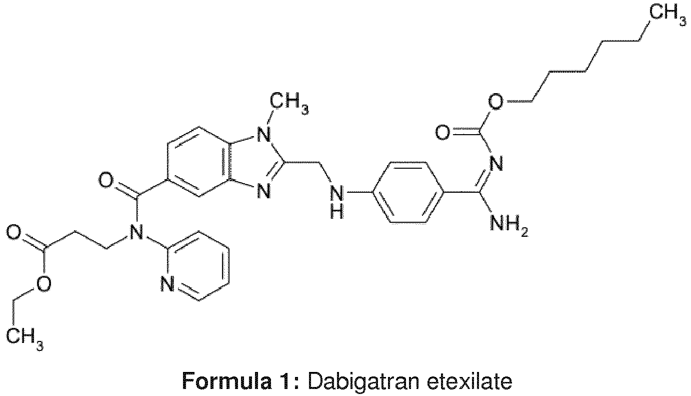

Dabigatran etexilate was approved by the U.S. Food and Drug Administration (FDA) for reducing the risks of stroke or systemic embolism in patients with non-valvular atrial fibrillation. Currently, the approved dosage form is capsules with strengths of 75 mg, 110 mg and 150 mg, and the product name is Pradaxa. It is disclosed in EP1870100, wherein also disclosed, pellet formulation of dabigatran etexilate methanesulfonate. This composition is formulated with a core material consisting of organic acid and an active layer which encloses the core. Each PRADAXA® capsule contains the following inactive ingredients: acacia, dimethicone, hypromellose, hydroxypropylcellulose, tartaric acid, carrageenan, potassium chloride, talc, titanium dioxide, and gelatin.

In prior art, the most of formulations are in the form of capsule and tablet. However, many of the formulations in the art rely on complex formulation which can add to the cost of the manufacture of the drug or can be subject to malfunction leading to incorrect or inappropriate administration of the drug.

The solubility of dabigatran etexilate in water is only 1.2 mg/ml. Moreover, the active ingredient has a strong pH-dependent solubility that is greatly increased in the acidic environment. This leads to the problem that conventional oral pharmaceutical formulations have large variations in the bioavailability since the solubility of the active ingredient depends on the pH value in the patient's stomach. This is particularly problematic with patients in whom the stomach pH value is changed by physiological variability, illness, or pre-medications (for example, proton pump inhibitors). There is therefore a need for oral pharmaceutical formulations of dabigatran etexilate.

In this invention, organic acid is used to provide a release that is independent from the pH value of the stomach and thus, provide solubility of the active ingredient and bioavailability.

Dabigatran etexilate has incompatibility problem and stability problem when it is combined with acid. We have found an easy way to separate the organic acid from the dabigatran etexilate formulation. Multi-layered tablet is used so this overcomes the problems. It also provides improvement in dissolution profile. Thus, the organic acid and dabigatran etexilate did not interact with each other, also the tablet provides easy and cost-effective process.

Furthermore, in this invention, the choice of suitable excipients in each layer and using certain amounts of them are important to ensure good solubility without the addition of significant side effect.

### Detailed Description of the Invention

The main object of the present invention is to avoid incompatibility problems and stability problems between active agent and acid by using multilayer tablet, especially bilayer tablet and to provide an easy and cost-effective process for the preparation of the said pharmaceutical formulation.

Another object of the present invention is to provide desired dissolution profiles in tablet, desired bioavailability and a long shelf life by the help of selection of excipients in a certain ratio.

Absorption of the active substances generally occurs in the small intestine due to a large surface area and the slow peristaltic movements of the small intestine that has a basic media. However, absorption from the stomach that has an acidic media is negligibly low due to the fast peristaltic movements and the high the surface area of the stomach. Thus, the dissolution of the active substances in the small intestine, a basic media, is important. Dabigatran, is weakly basic, should be dissolved in acidic environments and the dissolution of dabigatran should be pH independent. These problems can be solved by the addition of an organic acidic or inorganic acid.

In one embodiment, organic acid is used.

Accordingly, the selected organic acid keeps the pH of the micro-environment low, for a long time whilst remain undissolved in tablets or capsules and increases the solubility of the active agent. Also, organic acid aids the spheronisation of the pellets due to lubricant property of organic acid and contributes the production of fine pellets. By means of organic acid, the surface area of the obtained fine pellets is increased and as a result the content uniformity of the formulation is increased.

In one embodiment, organic acid is in pellet form.

In this invention, organic acid pellets are used. Thus, the present invention relates to an oral pharmaceutical formulation comprising dabigatran etexilate or a pharmaceutically acceptable salt thereof and organic acid pellets.

Multi-layered tablets are known as a novel drug delivery system. Compaction of different granules in the form of various layer in single tablets are called as multi-layered tablets. It generally consists of parallel, clear, colored, visual distinct layers two to three or more APIs or APIs along with functional or non-functional placebo layers. Multi-layered tablet dosage forms are designed for a variety of reasons such as incompatibility problems between active agents or excipients.

Dabigatran etexilate has a stability problem when it is combined with acid. Multi-layered tablet is used, so this overcomes the problems. Thus, the acid and dabigatran did not interact with each other, also this provides easy and cost-effective process. In other words, this problem can be overcome by means of a bilayer pharmaceutical tablet comprising the first layer having dabigatran etexilate prepared from a pharmaceutical formulation mentioned hereinbefore under the first aspect of the invention and a second layer having organic acid pellets.

In this invention, the pharmaceutical bilayer tablet comprises;
a. A first layer having dabigatran etexilate free base or pharmaceutically acceptable salts of dabigatran etexilate and at least one pharmaceutically acceptable excipient
b. A second layer having organic acid pellets and at least one pharmaceutically acceptable excipient, wherein the weight ratio of the second layer to the first layer is between 0.1 and 4.0, preferably the weight ratio of the second layer to the first layer is between 0.4 and 2.0.

The ratio of the second layer to the first layer is important to ensure desired stability and desired dissolution profiles without the addition of significant side effect.

In this invention, the amount of dabigatran etexilate free base or pharmaceutically acceptable salts of dabigatran etexilate in the first layer is 20.0% to 70.0% by weight, preferably 30.0% to 50.0% by weight and the amount of organic acid pellet in the second layer is 20.0% to 70.0% by weight, preferably 30.0% to 50.0% by weight.

In another embodiment, the weight ratio of organic acid pellet in the second layer to dabigatran etexilate free base or pharmaceutically acceptable salts of dabigatran etexilate in the first layer is 0.1-10.0, preferably the ratio is 0.2-5.0.

In another embodiment, there is provided a pharmaceutical formulation wherein the first layer is substantially free of an organic acid pellet.

Suitable organic acid pellets are comprising at least one carboxylic group. They are selected from the group comprising citric acid, tartaric acid, gallic acid, orotic acid, p-coumaric acid, hippuric acid, ferulic acid, vanillic acid, fumaric acid, maleic acid, succinic acid, malic acid, glutamic acid, aspartic acid, oxalic acid, lactic acid, formic acid, acetic acid, propionic acid, caproic acid, benzoic acid, carbonic acid or mixtures thereof.

According to one embodiment of the present invention, preferably the organic acid is citric acid or tartaric acid or mixtures thereof.

The reproducibility of the pellet formulations is also much better than the reproducibility of the single-unit dosage forms. They are suitable systems for film coating with respect to the low surface area-volume ratios. Also, one of the advantageous properties of the pellet formulations is being good resistance to external factors such as moisture, air and light.

In one embodiment, the organic acid pellets are coated with isolation solution.

In one embodiment, isolation solution is formed of a polymeric or a non-polymeric pharmaceutically acceptable agent or mixtures thereof.

According to one embodiment of this present invention, the pharmaceutically acceptable excipient in each layer is selected from disintegrants, lubricants, glidants, binders, fillers or mixtures thereof.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active ingredient and the excipients.

In this invention, each layer comprises at least one same excipient.

A further advantage of the present invention is to provide less side effects. It has been found that during the process of a bilayer tablet of dabigatran etexilate, same excipient has been used for each layer. Also, this helps to achieve an effective and easy process.

The selection of excipients has more importance to obtain the ideal disintegrating time during the shelf life. Especially, the choice of the disintegrant has a major role in the manufacture of the tablets since it has an impact on both hardness and mouth feel of the formulation. Therefore, the choice of a suitable disintegrant and an optimal use level is critical to ensure a high disintegration rate.

Suitable disintegrants are selected from a group comprising croscarmellose sodium, microcrystalline cellulose, starch, sodium starch glycolate, crospovidone (cross-linked polyvinil pyrrolidone), povidone, poloxamer, low-substituted hydroxypropyl cellulose, pregelatinized starch, sodium carboxymethyl cellulose, calcium carboxymethyl cellulose, carboxymethyl cellulose, docusate sodium, guar gum, polyacryline potassium, sodium alginate, alginic acid, alginates, ion-exchange resins, magnesium aluminium silica, sodium glycine carbonate, sodium lauryl sulphate or mixtures thereof.

According to one embodiment of the present invention, preferably the disintegrant is croscarmellose sodium and it is used in each layer.

In the present invention, the amount of croscarmellose sodium is between 1.0% and 15.0% by weight of each layer.

In the present invention, the pharmaceutical bilayer tablet of dabigatran comprises croscarmellose sodium as disintegrant, dissolution problems are solved and surprisingly better dissolution profile is gained.

Suitable lubricants are selected from a group comprising magnesium stearate, sodium stearyl fumarate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium lauryl sulphate or mixtures thereof.

According to one embodiment of the present invention, preferably the lubricant is magnesium stearate and it is used in each layer.

In the present invention, the amount of lubricant is between 0.1% and 5.0% by weight of each layer.

Suitable glidants are selected from a group comprising colloidal silicon dioxide, talc, aluminium silicate or mixtures thereof.

According to one embodiment of the present invention, preferably the glidant is colloidal silicon dioxide and it is used in each layer.

In the present invention, the amount of glidant is between 0.5% and 3.0% by weight of each layer.

Suitable binders are selected from the group comprising hydroxypropyl methyl cellulose, pregelatinized starch, povidone, copovidone, copolyvidone, polyvinylpyrrolidone, carnauba wax, , pullulan, polymethacrylate, glyceryl behenate, hydroxypropyl cellulose, carboxymethyl cellulose, methyl cellulose, hydroxyethyl cellulose, sodium carboxymethyl cellulose, carboxymethyl cellulose calcium, ethyl cellulose, microcrystalline cellulose, polymetacrylates, polyethylene oxide, polyvinyl alcohol, polycarbophil, polyvinyl acetate and its copolymers, gelatin, starch, xanthan gum, guar gum, alginate, carrageen, kollagen, agar, pectin, hyaluronic acid, carbomer, cellulose acetate phthalate, hydroxypropyl starch, hydroxyethyl methyl cellulose, poloxamer, polyethylene glycol, sugars, natural gums, polyacrylamide, aluminum hydroxide, benthonite, laponite, setostearyl alcohol, polyoxyethylene-alkyl ethers, polydextrose or mixtures thereof.

According to one embodiment of the present invention, preferably the binder is hydroxypropyl methyl cellulose and it is used in each layer.

In the present invention, the amount of binder is between 10.0% and 30.0% by weight of each layer.

Suitable fillers are selected from a group comprising microcrystalline cellulose, lactose, mannitol, spray-dried mannitol, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate, polyols, dextrose, maltitol or mixtures thereof.

According to one embodiment of the present invention, preferably the filler is microcrystalline cellulose and it is used in each layer.

In the present invention, the amount of filler is between 10.0% to 50.0% by weight of each layer.

According on this embodiment of the invention, calculation is done as the percentiles for each layer separately. The pharmaceutical bilayer tablet comprises;

### First layer:

20.0-70.0% by weight of dabigatran etexilate free base or pharmaceutically acceptable salts thereof
10.0-50.0% by weight of microcrystalline cellulose
10.0-30.0% by weight of hydroxypropyl methyl cellulose
0.5-3.0% by weight of colloidal silicon dioxide
1.0-15.0% by weight of croscarmellose sodium
0.1-5.0% by weight of magnesium stearate

### Second layer:

20.0-70.0% by weight of isolated organic acid pellets
10.0-50.0% by weight of microcrystalline cellulose
10.0-30.0% by weight of hydroxypropyl methyl cellulose
0.5-3.0% by weight of colloidal silicon dioxide
1.0-15.0% by weight of croscarmellose sodium
0.1-5.0% by weight of magnesium stearate

The tablets may be prepared by a granulation process. A characteristic of the methods given below based on wet granulation and used for the production of the formulations of the invention. Suitable granulation solutions are selected from a group comprising pure water, ethyl alcohol, glycerin, sorbitol, polyethylene glycol, propylene glycol, isopropyl alcohol, or mixtures thereof, preferably granulation solution is pure water.

According to whole embodiments of this present invention, a stable, a cost-effective, an easy to prepare process and a desired in vitro release,an improved dissolution profile and a desired bioavailability of dabigatran etexilate is achieved.

Direct compression method is the most commonly used method in producing tablet because it is the easiest method in tablet manufacturing, can use conventional manufacturing instrument, short procedure, relatively cheap, can be loaded with thermolabile and moisture sensitive drugs and can be made into high dose.

According to another embodiment of this present invention, the tablet is coated with film coating. The film coating comprises coating agents.

Suitable coating agent are selected from the group comprising polyvinyl alcohol (PVA), talc, polymethacrylates, hydroxypropyl methylcellulose, sodium lauryl sulfate, glyceryl monocaprylocaprate, lactose monohydrate, hydroxypropyl cellulose, polyethylene glycol (PEG), polyvinyl alcohol-polyethylene glycol copolymers, ethylcellulose dispersions, polyvinylprolidone, polyvinylprolidone-vinyl acetate copolymer (PVP-VA), all kinds of Opadry®, pigments, dyes, titanium dioxide, macrogol, coloring agent or mixtures thereof.

Suitable coloring agents are selected from the group comprising ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

The process for preparation of the pharmaceutical bilayer tablet comprises the following steps:
a. first layer
   - weighing, sieving and mixing microcrystalline cellulose, hydroxypropyl methyl cellulose, colloidal silicon dioxide, croscarmellose sodium and dabigatran etexilate free base or pharmaceutically acceptable salt of dabigatran etexilate
   - granulating the powder mixture with water
   - drying in a vacuum oven at 50-55°C and sieving.
   - adding magnesium stearate to the mixture and mixing.
b. second layer
   - weighing, sieving and mixing microcrystalline cellulose, hydroxypropyl methyl cellulose, colloidal silicon dioxide, croscarmellose sodium.
   - granulating the powder mixture with water
   - drying in a vacuum oven at 50-55°C
   - adding isolated organic acid pellets to the mixture and mixing.
   - adding magnesium stearate to the mixture and mixing.
c. Then, pressing layers to form bilayer tablets,

Furthermore, the tablet may be coated with a film coating.

### Example 1: Bilayer tablet

| **First Layer** | **(%) amount (w/w)** |
|---|---|
| Dabigatran etexilate free base or pharmaceutically acceptable salts of dabigatran etexilate | 25.0%-60.0% |
| Microcrystalline cellulose | 15.0%-40.0% |
| Hydroxypropyl methyl cellulose | 12.0%-27.0% |
| Colloidal silicon dioxide | 0.6%- 2.5% |
| Croscarmellose sodium | 2.0%-12.0% |
| Magnesium stearate | 0.2%-4.0% |
| **Total (First layer)** | **100** |

| **Second Layer** | **(%) amount (w/w)** |
|---|---|
| Isolated organic acid pellets | 25.0%-60.0% |
| Microcrystalline cellulose | 15.0%-40.0% |
| Hydroxypropyl methyl cellulose | 12.0%-27.0% |
| Colloidal silicon dioxide | 0.6%- 2.5% |
| Croscarmellose sodium | 2.0%-12.0% |
| Magnesium stearate | 0.2%-4.0% |
| **Total (Second Layer)** | **100** |

The process for preparation of the pharmaceutical bilayer tablet comprises the following steps:
a. first layer
   - weighing, sieving and mixing microcrystalline cellulose, hydroxypropyl methyl cellulose, colloidal silicon dioxide, croscarmellose sodium and dabigatran etexilate free base or pharmaceutically acceptable salt of dabigatran etexilate
   - granulating the powder mixture with water
   - drying in a vacuum oven at 50-55°C and sieving.
   - adding magnesium stearate to the mixture and mixing.
b. second layer
   - weighing, sieving and mixing microcrystalline cellulose, hydroxypropyl methyl cellulose, colloidal silicon dioxide, croscarmellose sodium.
   - granulating the powder mixture with water
   - drying in a vacuum oven at 50-55°C
   - adding isolated organic acid pellet to the mixture and mixing.
   - adding magnesium stearate to the mixture and mixing.
c. then, pressing layers to form bilayer tablets,
d. optionally, coating the bilayer tablet with film coating

### Example 2: Bilayer tablet

| **First Layer** | **(%) amount (w/w)** |
|---|---|
| Dabigatran etexilate free base or pharmaceutically acceptable salts of dabigatran etexilate | 30.0%-50.0% |
| Microcrystalline cellulose | 20.0%-30.0% |
| Hydroxypropyl methyl cellulose | 15.0%-25.0% |
| Colloidal silicon dioxide | 0.7%- 2.0% |
| Croscarmellose sodium | 3.0%-10.0% |
| Magnesium stearate | 0.2%-3.0% |

| **Total (First layer)** | **100** |
|---|---|
| **Second Layer** | **(%) amount (w/w)** |
| Isolated organic acid pellets | 30.0%-50.0% |
| Microcrystalline cellulose | 20.0%-30.0% |
| Hydroxypropyl methyl cellulose | 15.0%-25.0% |
| Colloidal silicon dioxide | 0.7%- 2.0% |
| Croscarmellose sodium | 3.0%-10.0% |
| Magnesium stearate | 0.2%-3.0% |
| **Total (Second Layer)** | **100** |

The process for preparation of the pharmaceutical bilayer tablet comprises the following steps:
a. first layer
   - weighing, sieving and mixing microcrystalline cellulose, hydroxypropyl methyl cellulose, colloidal silicon dioxide, croscarmellose sodium and dabigatran etexilate free base or pharmaceutically acceptable salt of dabigatran etexilate
   - granulating the powder mixture with water
   - drying in a vacuum oven at 50-55°C and sieving.
   - adding magnesium stearate to the mixture and mixing.
b. second layer
   - weighing, sieving and mixing microcrystalline cellulose, hydroxypropyl methyl cellulose, colloidal silicon dioxide, croscarmellose sodium.
   - granulating the powder mixture with water
   - drying in a vacuum oven at 50-55°C
   - adding isolated organic acid pellet to the mixture and mixing.
   - adding magnesium stearate to the mixture and mixing.
c. then, pressing layers to form bilayer tablets,
d. optionally, coating the bilayer tablet with film coating

### Preparation of Isolated Organic Acid Pellets (Coated Organic Acid Pellets):

### Preparation isolation solution:

### Formula 1:

HPMC and sucrose are added to purified water and mixed. Talc is added to the obtained mixture and mixed.

### Formula 2:

HPMC and triethyl citrate are added to purified water and mixed. Talc is added to the obtained mixture and mixed.

### Formula 3:

HPMC is added to purified water and mixed in homogenisator. Talc and PEG 6000 are added to the obtained mixture and mixed in homogenisator.

Organic acid pellets are coated with isolation solution which is selected from formula 1, formula 2 or formula 3.

The preparation process of the invention is simple and suitable for industrial production.

## Claims

1. A pharmaceutical bilayer tablet comprising;
a. A first layer having dabigatran etexilate free base or pharmaceutically acceptable salts of dabigatran etexilate and at least one pharmaceutically acceptable excipient,
b. A second layer having organic acid pellets and at least one pharmaceutically acceptable excipient,
wherein the weight ratio of the second layer to the first layer is between 0.1 and 4.0.

2. The pharmaceutical bilayer tablet according to claim 1, wherein the weight ratio of the second layer to the first layer is between 0.4 and 2.0.

3. The pharmaceutical bilayer tablet according to claim 1, wherein the weight ratio of organic acid pellets in the second layer to dabigatran etexilate free base or pharmaceutically acceptable salts of dabigatran etexilate in the first layer is 0.1-10.0, preferably the ratio is 0.2-5.0.

4. The pharmaceutical bilayer tablet according to claim 3, wherein the amount of dabigatran etexilate free base or pharmaceutically acceptable salts of dabigatran etexilate in the first layer is 20.0% to 70.0% by weight and the amount of organic acid pellets in the second layer is 20.0% to 70.0% by weight.

5. The pharmaceutical bilayer tablet according to claim 4, wherein the amount of dabigatran etexilate free base or pharmaceutically acceptable salts of dabigatran etexilate in the first layer is 30.0% to 50.0% by weight and the amount of organic acid pellets in the second layer is 30.0% to 50.0% by weight.

6. The pharmaceutical bilayer tablet according to claim 1, wherein the first layer is free of an organic acid pellet.

7. The pharmaceutical bilayer tablet according to any preceding claims, wherein said organic acid pellets are selected from the group comprising citric acid, tartaric acid, gallic acid, orotic acid, p-coumaric acid, hippuric acid, ferulic acid, vanillic acid, fumaric acid, maleic acid, succinic acid, malic acid, glutamic acid, aspartic acid, oxalic acid, lactic acid, formic acid, acetic acid, propionic acid, caproic acid, benzoic acid, carbonic acid or mixtures thereof.

8. The pharmaceutical bilayer tablet according to claim 7, wherein said organic acid pellets are citric acid or tartaric acid or mixtures thereof.

9. The pharmaceutical bilayer tablet according to claim 8, wherein organic acid pellets are coated isolating solution.

10. The pharmaceutical bilayer tablet according to claim 9, wherein isolation solution is formed of a polymeric or a non-polymeric pharmaceutically acceptable agent or mixtures thereof.

11. The pharmaceutical bilayer tablet according any preceeding claims, wherein at least one pharmaceutically acceptable excipient in each layer is selected from disintegrants, lubricants, glidants, binders, fillers or mixtures thereof.

12. The pharmaceutical bilayer tablet according to claim 11, wherein each layer comprising at least one same excipient.

13. The pharmaceutical bilayer tablet according to claim 11, wherein the disintegrants in each layer are selected from a group comprising croscarmellose sodium, microcrystalline cellulose, starch, sodium starch glycolate, crospovidone, povidone, poloxamer, low-substituted hydroxypropyl cellulose, pregelatinized starch, sodium carboxymethyl cellulose, calcium carboxymethyl cellulose, carboxymethyl cellulose, docusate sodium, guar gum, polyacryline potassium, sodium alginate, alginic acid, alginates, ion-exchange resins, magnesium aluminium silica, sodium glycine carbonate, sodium lauryl sulphate or mixtures thereof.

14. The pharmaceutical bilayer tablet according to claim 13, wherein the disintegrant is croscarmellose sodium and it is used in each layer.

15. The pharmaceutical bilayer tablet according to claim 14, wherein the amount of croscarmellose sodium is between 1.0% and 15.0% by weight of each layer.

16. The pharmaceutical bilayer tablet according to any preceding claims, wherein the bilayer tablet comprising;
a. first layer,
20.0-70.0% by weight of dabigatran etexilate free base or pharmaceutically acceptable salts thereof
10.0-50.0% by weight of microcrystalline cellulose
10.0-30.0% by weight of hydroxypropyl methyl cellulose
0.5-3.0% by weight of colloidal silicon dioxide
1.0-15.0% by weight of croscarmellose sodium
0.1-5.0% by weight of magnesium stearate of the amount of the first layer.
b. second layer,
20.0-70.0% by weight of isolated organic acid pellets
10.0-50.0% by weight of microcrystalline cellulose
10.0-30.0% by weight of hydroxypropyl methyl cellulose
0.5-3.0% by weight of colloidal silicon dioxide
1.0-15.0% by weight of croscarmellose sodium
0.1-5.0% by weight of magnesium stearate of the amount of the second layer.

17. The process for preparation of the pharmaceutical bilayer tablet according claim 16, wherein the process comprising the following steps:
a. first layer
- weighing, sieving and mixing microcrystalline cellulose, hydroxypropyl methyl cellulose, colloidal silicon dioxide, croscarmellose sodium and dabigatran etexilate free base or pharmaceutically acceptable salt of dabigatran etexilate
- granulating the powder mixture with water
- drying in a vacuum oven at 50-55°C and sieving.
- adding magnesium stearate to the mixture and mixing.
b. second layer
- weighing, sieving and mixing microcrystalline cellulose, hydroxypropyl methyl cellulose, colloidal silicon dioxide, croscarmellose sodium.
- granulating the powder mixture with water
- drying in a vacuum oven at 50-55°C
- adding isolated organic acid to the mixture and mixing.
- adding magnesium stearate to the mixture and mixing.
c. then, pressing layers to form bilayer tablets.
d. optionally, coating the bilayer tablet with film coating
